Europäisches Patentamt

European Patent Office

Office européen des brevets

(11) Publication number: **0 409 180 A1**

## (12) EUROPEAN PATENT APPLICATION

(21) Application number: 90113698.6

(22) Date of filing: 17.07.90

(51) Int. Cl.5: **A61M 5/32**

(30) Priority: 21.07.89 ES 8902593

(43) Date of publication of application:
**23.01.91 Bulletin 91/04**

(84) Designated Contracting States:
**AT BE CH DE DK ES FR GB GR IT LI LU NL SE**

(71) Applicant: COMERCIAL MARIAE, S.L.
Rio Ega, 46
E-31005 Pamplona(ES)

Applicant: Sempere Escudero, Philippe
Aragon, 41
E-08015 Barcelona(ES)

(72) Inventor: Sempere Escudero, Philippe
Aragon, 41
E-08015 Barcelona(ES)

(74) Representative: Duran Moya, Luis-Alfonso
c/o DURAN-CORRETJER Paseo de Gracia no.
101
E-08008 Barcelona(ES)

(54) **A safety protective device for injection needles.**

(57) A safety protective device for injection needles
of the type which consists of a minimum of two
bodies (1,2) related telescopically, enveloping the
needle (7) which is attached firmly to one of them,
that being the body that is provided with a housing
for lodging the injection syringe (4), there being a
compression spring (8) acting between the two bod-
ies (1,2) tending to separate them such that one of
the bodies encloses either partially or totally the
needle (7) depending on the position of a stop (44)
attached to one of the bodies and which moves
along a slot (41,42,45) of complex form in the other
body thus determining the specific position of the
axial stop (44).

FIG.7

FIG.1

# A SAFETY PROTECTIVE DEVICE FOR INJECTION NEEDLES

## SPECIFICATION

This invention patent refers to safety device for the protection of injection needles, whether they be for hyperdermic, intramuscular or intravenous use.

The objective of the device for injection needles which is the object of this patent is to provide a protection of the needle by enclosing it automatically in tubular elements, thus ensuring that the needle is no longer usable for subsequent applications.

The fact that the injection needle, after being used is automatically protected in a blocked wrapping definitively limits the needle to one single application and hence avoids the possibilities of accidental wounding and exposure to contagion by a needle impregnated with dangerous residuals.

That the point of the needle once used is automatically protected simultaneous with its withdrawal from the patient's point of injection is without doubt a satisfactory assurance both for the safety of the professional himself and for the peace of mind of the patient.

The device for protecting and rendering the needle non-reusable, object of this patent is of special benefit given the diversity of needles currently used, and bearing in mind the considerable increase in the number of people who are centres of contagion due to the re-utilization of needles and in particular the problems associated with the transmission of AIDS and Hepatitis.

Essentially the device, object of this patent is of the type that is based on the enclosure of the needle within an assembly of two or more bodies which can be displaced telescopically one within the other against the reaction of a spring fitted within the assembly which tends to separate the bodies, there being however an initial equilibrium position in which the needle which is attached to an internal salient of the outer body protrudes slightly from the second body to permit its insertion, also a second position at which the said second body after having given effect to the application of the needle and by the action of the spring reaches a position of blockage where the needle is completely enveloped making reutilization quite impossible.

To accomplish its blocking function the enveloping body is provided with a system of complex slots and a mobile stop and these determine the position in use and in the post-use blocking position.

The accompanying drawings facilitate the understanding of the description showing an example of the injection needle object of this patent.

Figures 1, 2, 3, 4, show respectively longitudinal sections of a protective device of the type described in this patent, showing the positions respectively as supplied, in use to the completion of the insertion, and in the protected position. 4 shows a three body version in its telescopic disposition.

Figures 5, 6 and 7 are views of the specific examples of this patent, one in longitudinal section.

Figures 8 and 9 are perspective views of the double guide slot.

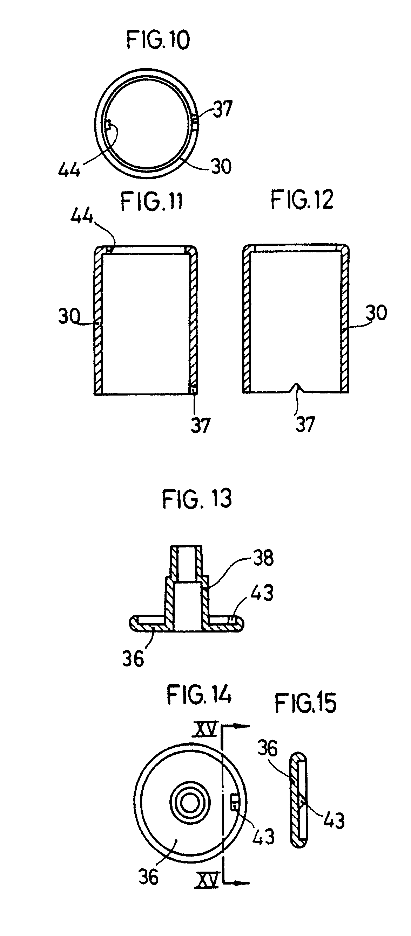

Figures 10, 11 and 12 are views of the enveloping body corresponding to figures 5 to 7.

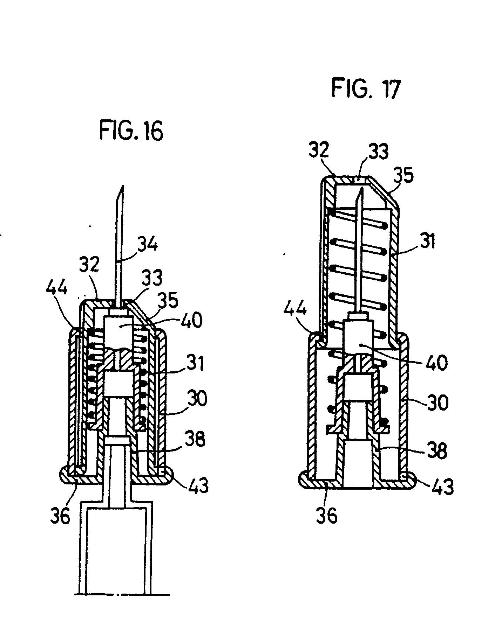

Figures 13, 14 and 15 show the base component of the enveloping body corresponding to the example shown in figures 5 to 7.

Figures 16 and 17 are sectional drawings showing the position respectively in use and in the subsequent blocked state.

Figures 18 and 19 show the variant with an extra long needle and the double telescopic assembly.

The device which is the object of this patent belongs to the type, as shown by the drawings, that comprises a system consisting of two bodies -1- and -2- fitted together in telescopic assembly and in which the outer body -1- has at one extremity an orifice which can accept the body -2- with capacity for its movement. This same body -1- has, at the extremity opposed to that mentioned above a cavity -3- which can house the extremity of the syringe vessel -4-. The body -1- also has an internal projection -5- with an axial orifice -6- for the passage of liquid and which carries the properly so called injection needle -7-.

A fine compression spring -8- tends to separate the two bodies -1- and -2-.

An external cover -9- closes the assembly for purposes of storage and transport.

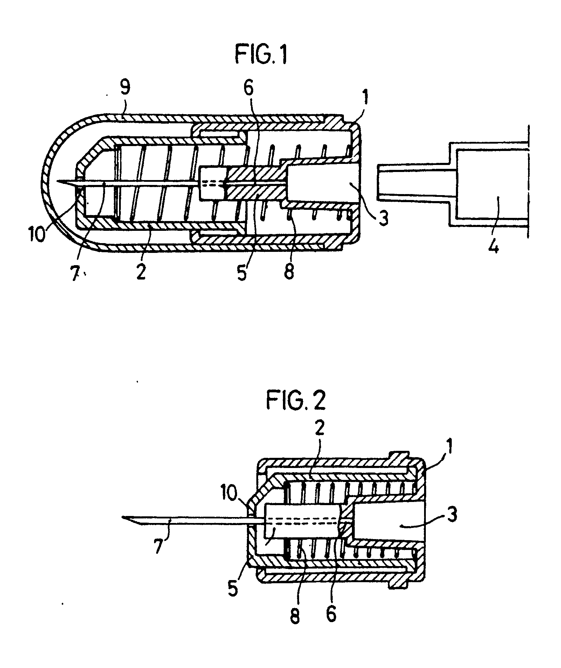

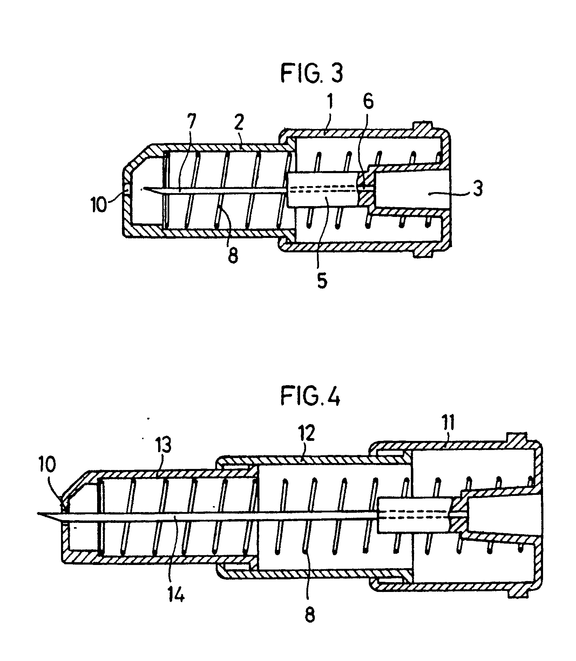

In its utilization state the sharp end of the needle -7- protrudes through the orifice -10- on the front face of the sliding body -2- such that after the insertion of the needle in the body of the patient the state of the components is as is shown in figure 2, that is with the body -2- completely introduced into the interior of body -1- and the needle -7- in a fully visible or salient position. Following this application phase the internal spring -8- causes one body component to be displaced with respect to the other arriving at the state shown in figure 3 in which the telescopic body -2- surrounds and encloses fully the needle -7-.

For extra long needles the arrangement is as shown in figure 4, with its three successive bodies -11-, -12- and 13 disposed in a manner analogous to to that shown previously with the needle -14- having its 10. greater length protected in the same manner as shown above but with the involvement of two sliding bodies -12- and -13-.

The principle of operation of the device which is the object of this patent is based on the provision of two stable positions for the external protector body -2-, the first being as shown in figure 1 and corresponds to a stopping means locating so that the sliding body -2-, under the action of hte spring -8- cannot fully cover the needle.

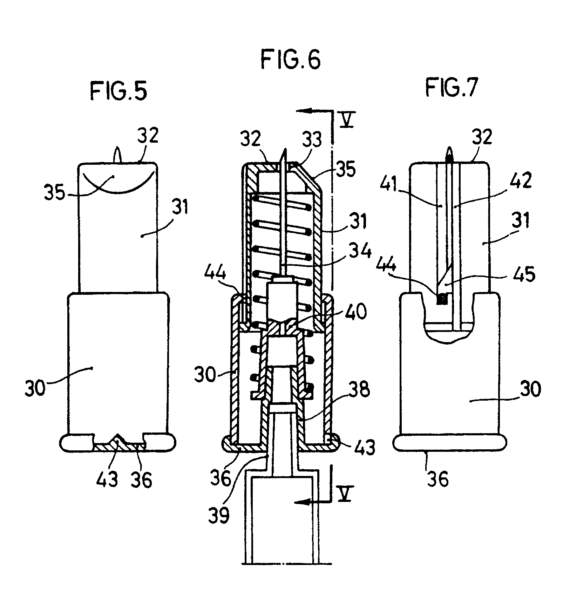

The means provided in the present invention comprise what is shown in figures 5 and successive figures. These include an enveloping body -30- and a telescopic component -31- having a flat extremity -32- in which is the orifice -33- for the issue of the needle -34- and a chamfered area -35-. The body -30- is attached to a base -36- which has a small projection -43- which corresponds to a notch -37- in the body -30- for purpose of centralizing the base -36-. The base -36- has centrally in its interior a housing -38- to receive the extremity of the syringe -39-.

The needle -34- is attached to a hood -40- lodged at the extremity of the housing -38- which projects from the above mentioned base -36-.

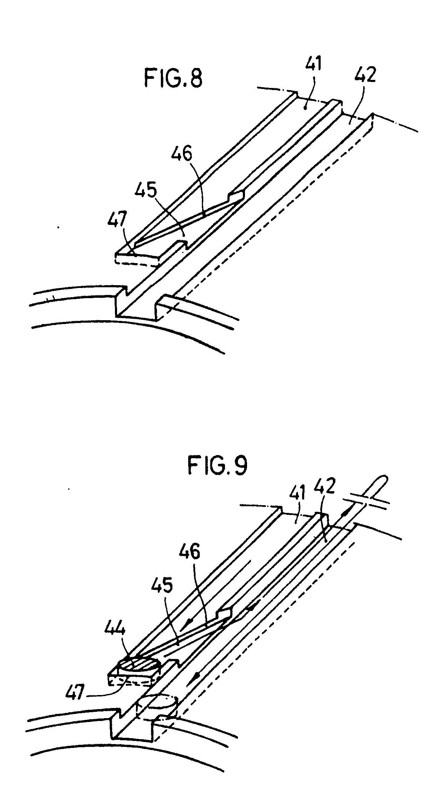

The double slot for guiding the slot component consists, as may be seen in the figure 8 and 9, of two parallel grooves -41- and -42- the second of the two being deeper and longer than the first so that the stop -44- which is located on the edge of the upper opening of the telescopic body -30- may fulfil its function of the final blocking in accordance with this invention.

The functioning of the stop in conjunction with the double slot requires also that the groove -41- has a deeper zone -45- at its extremity with an obliquely angled edge -46- and an end retaining wall -47-. By means of this layout the stop -44- is located as may be seen in full line in figure 9 corresponding to the position when the needle is supplied. This stop -44-follows along the length of the limiting face -46- when the needle is used such that it reaches, as the telescopic body -31- is turned the slot -42-, attaining the position of retention shown in dotted line in figure 9, at which stage the telescopic body can no longer be displaced being prevented by the compression spring.

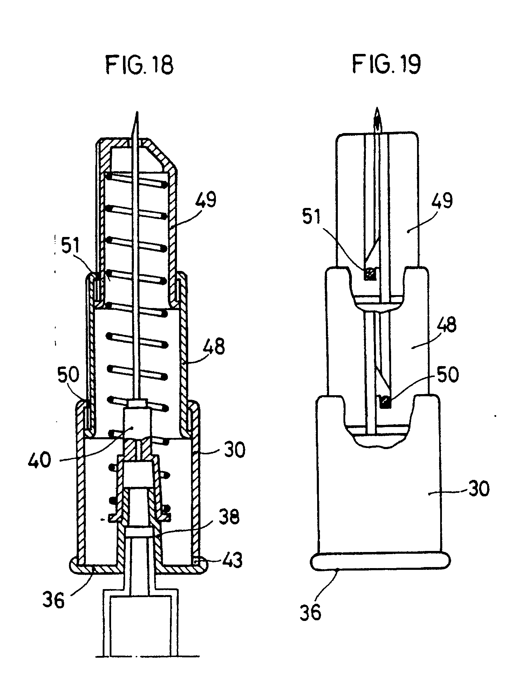

Figures 18 and 19 show the device with two telescopic sections -48- and -49- successively and each each with its system of double Slots and stop as has been explained above but with the single difference that one of the mentioned systems of slots is inverted in position as can be seen in figure 19, showing the respective stops -50- and -51- on the corresponding bodies -48- and -49-.

Anything not affecting, altering or modifying the essence of the described device is a variable for purpose of htis patent.

## Claims

1.- A safety protective device for injection needles of the type which consists of a minimum of two bodies related telescopically, enveloping the needle which is attached firmly to one of them, that one being the body that is provided with a housing for lodging the injection syringe, there being a comression spring acting between the two bodies tending to separate them such that one of the bodies encloses either partially or totally the needle depending on the position of a stop attached to one of the bodies and which moves along a slot of complex form in the other body this determining the specific position of the axial stop, characterized in that the stop consists of a small protrusion on the upper edge of the open end of the fixed envelopping body that at its other extremity has the syringe connected thereto.

2.- A safety protective device for injection needles as in claim 1 characterized in that the complex slot guiding the stop comprises a double slot of parallel grooves one of which is shallower terminating in a lower zone and an end wall to arrest the stop in its position for supplying the needle and having an oblique angled step separating the two mentioned zones and forming a communicating area between the lower zone and the second groove, laterally, the second slot being of greater length.

3.- A safety protective device for injection needles as in claim 1, characterized in that the enclosing body which receives the syringe comprises the cylindrical enveloping component having on its lower edge a centering notch, a flange cover for holding the said cylindrical component and which has a protrusion correspoding to the notch for centering both pieces as well as a hollow internal prolongation which houses the syringe and on its outer surface carries the sleeve of the needle.

## FIG.1

## FIG.2

## FIG. 3

## FIG.4

FIG.5　　FIG.6　　FIG.7

FIG.8

FIG.9

FIG.10

FIG.11  FIG.12

FIG. 13

FIG.14  FIG.15

FIG. 16

FIG. 17

FIG. 18

FIG. 19

European
Patent Office

**EUROPEAN SEARCH
REPORT**

Application Number

**EP 90 11 3698**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 802 638 (WANDERER et al.) <br> * Figures 28,29,31,32; page 25, lines 7-10; page 25, line 22 - page 26, line 6; page 27, lines 10-24 * <br> – – – | 1,2 | A 61 M 5/32 |
| A | EP-A-0 268 445 (STERIMATIC HOLDINGS LTD) <br> * Figures 1-3; column 2, lines 14-17; column 3, lines 24-51 * <br> – – – – – | 1,2 | |
| | | | TECHNICAL FIELDS SEARCHED (Int. Cl.5) |
| | | | A 61 M |

The present search report has been drawn up for all claims

| Place of search | Date of completion of search | Examiner |
|---|---|---|
| The Hague | 29 October 90 | SEDY, R. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

-------------------------------------------------------------

& : member of the same patent family, corresponding
document